Europäisches Patentamt

European Patent Office

Office européen des brevets

(19)

(11) Veröffentlichungsnummer: **0 005 788**
**B1**

(12) **EUROPÄISCHE PATENTSCHRIFT**

(21) Anmeldenummer: **79101555.5**

(51) Int. Cl.³: **C 07 C 11/167, C 07 C 7/08**

(22) Anmeldetag: **22.05.79**

(54) Verfahren zur Gewinnung von Butadien-1,3 aus einem C4-Kohlenwasserstoffgemisch

(30) Priorität: **01.06.78 DE 2823983**

(43) Veröffentlichungstag der Anmeldung:
**12.12.79 Patentblatt 79/25**

(45) Bekanntmachung des Hinweises auf die Patenterteilung:
**21.01.81 Patentblatt 81/03**

(84) Benannte Vertragsstaaten:
**BE DE FR GB IT NL**

(56) Entgegenhaltungen:
**DE - A - 2 251 051**
**US - A - 2 486 929**
**US - A - 3 620 930**
**DE - A - 2 251 051**

(73) Patentinhaber: **BASF Aktiengesellschaft**
**Carl-Bosch-Strasse 38**
**D - 6700 Ludwigshafen (DE)**

(72) Erfinder: **Lindner, Alfred, Dr.**
**Ringstrasse 22**
**D - 6712 Bobenheim-Roxheim 1 (DE)**
**Volkamer, Klaus, Dr.**
**Heidelberger Ring 21**
**D - 6710 Frankenthal (DE)**
**Wagner, Ulrich, Dr.**
**Knospstrasse 7**
**D - 6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Gewinnung von Butadien-1,3 aus einem C$_4$-Kohlenwasserstoffgemisch

Die vorliegende Erfindung betrifft ein Verfahren zur Gewinnung von Butadien-1,3 aus einem Butadien-1,3 und kleine Mengen Propin enthaltenden C$_4$-Kohlenwasserstoffgemisch.

Es ist bekannt, z.B. aus der GB—PS 1 378 385, Butadien-1,3 aus einem Butadien-1,3 und kleine Mengen Propin enthaltenden C$_4$-Kohlenwasserstoffgemisch in der Weise zu gewinnen, daß das C$_4$-Kohlenwasserstoffgemisch durch extraktive Destillation mit einem selektiven Lösungsmittel aufgetrennt wird, wobei ein Butadien-1,3 erhalten wird, das noch einen großen Teil des im Ausgangs-C$_4$-Kohlenwasserstoffgemisch enthaltenen Propins enthält. Das Propin enthaltende Butadien-1,3 wird daher zur Abtrennung des Propins noch einer Destillation unterworfen. Wegen der Neigung des Propins zur Selbstzersetzung kann jedoch aus Sicherheitsgründen bei der Destillation des Propin enthaltenden Butadien-1,3 nur ein durch Butadien-1,3 verdünntes Propin-Destillat mit einem Propingehalt von etwa 50 Vol% abgezogen werden, was zu erheblichen Butadien-1,3-Verlusten führt. Um die Butadien-1,3-Verluste zu vermindern, wird bei dem bekannten Verfahren das mit Butadien-1,3 verdünnte Propin-Destillat in den Destillationsteil einer Äthylenanlage zurückgeführt.

Dieses Verfahren ist jedoch nicht befriedigend, zumal es nur durchführbar ist, wenn in der Nähe der Butadienanlage eine Äthylenanlage zur Verfügung steht. Eine andere Möglichkeit zur Abtrennung des Propins besteht darin, daß das kleine Mengen Propin enthaltende Ausgangs-C$_4$-Kohlenwasserstoffgemisch vor der extraktiven Destillation einer Destillation unterzogen wird, bei der ein durch C$_3$- und C$_4$-Kohlenwasserstoffe verdünntes Propin-Destillat abgezogen wird. Bei dieser Arbeitsweise sind eine hohe Sumpftemperatur und ein hoher Betriebsdruck erforderlich, um mit Kühlwasser am Kopf der Kolonne kondensieren zu können. Eine hohe Sumpftemperatur begünstigt jedoch die thermische Dimerisation des Butadiens und die Polymerisation.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Gewinnung von Butadien-1,3 mit Hilfe eines selektiven Lösungsmittels aus einem C$_4$-Kohlenwasserstoffgemisch, welches Butadien-1,3, Propin, im selektiven Lösungsmittel löslichere Kohlenwasserstoffe als Butadien-1,3 und im selektiven Lösungsmittel weniger lösliche Kohlenwasserstoffe als Butadien-1,3 enthält, bei dem das C$_4$-Kohlenwasserstoffgemisch unter Anwendung von einer oder mehreren extraktiven Destillationszonen in ein die weniger löslichen Kohlenwasserstoffe enthaltendes Destillat, einen Strom aus Butadien-1,3 und einen die löslicheren Kohlenwasserstoffe enthaltenden Strom aufgetrennt wird und das Propin entweder durch Destillation des C$_4$-Kohlenwasserstoffgemischs in einer der bzw. den extraktiven Destillationszonen vorgeschalteten Destillationszone oder durch Destillation des aus der bzw. den extraktiven Destillationszonen erhaltenen Butadien-1,3-Stromes in einer nachgeschalteten Destillationszone abgetrennt wird, wobei ein Propin und Butadien-1,3 enthaltender Kohlenwasserstoffstrom erhalten wird, welches dadurch gekennzeichnet ist, daß der Propin und Butadien-1,3 enthaltende Kohlenwasserstoffstrom einer weiteren Destillationszone zugeführt wird, wobei gleichzeitig ein zweiter Strom von flüssigen oder gasförmigen Kohlenwasserstoffen der weiteren Destillationszone zugeführt wird, und das Sumpfprodukt der Destillationszone in die extraktive Destillation zurückgeführt wird.

Nach dem neuen Verfahren ist es möglich, Butadien-1,3 in deutlich höherer Ausbeute aus einem Propin enthaltenden C$_4$-Kohlenwasserstoffgemisch als nach den bekannten Verfahren zu gewinnen, ohne daß ein erhöhtes Sicherheitsrisiko eingegangen werden muß. Außerdem kann die Destillationskolonne, aus der der Propin und Butadien-1,3 enthaltende Kohlenwasserstoffstrom erhalten wird, bei niedrigerer Sumpftemperatur betrieben werden, so daß die Polymerisationsgefahr vermindert wird.

Die erfindungsgemäß für die Gewinnung von Butadien-1,3 einzusetzenden, Propin enthaltenden C$_4$-Kohlenwasserstoffgemische werden z.B. bei der Herstellung von Äthylen und/oder Propylen durch thermisches Spalten einer Petroleumfraktion, z.B. von verflüssigtem Petroleumgas (LPG), Leichtbenzin (Naphtha), Gasöl und dergleichen, als Kohlenwasserstofffraktion erhalten. Weiterhin werden solche C$_4$-Fraktionen bei der katalytischen Dehydrierung von n-Butan und/oder n-Buten erhalten. Das C$_4$-Kohlenwasserstoffgemisch enthält in der Regel Butane, n-Buten, Isobuten, Butadien-1,3, Vinylacetylen, Äthylacetylen, Butadien-1,2, Propin und gegebenenfalls geringe Mengen an C$_5$-Kohlenwasserstoffen. Der Gehalt der C$_4$-Kohlenwasserstoffgemische an Propin liegt in der Regel zwischen 0,001 und 1 Gew.%, meistens unter 0,005 und 0,5 Gew.%. Das erfindungsgemäße Verfahren ist jedoch auch auf C$_4$-Kohlenwasserstoffgemische mit höheren Propin-Gehalten anwendbar, z.B. auf C$_4$-Kohlenwasserstoffgemische mit Propin-Gehalten von 0,001 bis 10 Gew.%, vorzugsweise 0,005 bis 5 Gew.%.

Als selektives Lösungsmittel kommen beispielsweise Carbonsäureamide, wie Dimethylformamid, Diäthylformamid, Dimethylacetamid, Formylmorpholin, Acetonitril, Furfurol, N-Methylpyrrolidon, Butyrolacton, Aceton, und ihre Mischungen mit Wasser in Betracht.

Mit besonderem Vorteil wird N-Methylpyrrolidon als selektives Lösungsmittel verwendet.

Im selektiven Lösungsmittel löslichere Kohlenwasserstoffe als Butadien-1,3 sind beispielsweise Vinylacetylen, Äthylacetylen, Butadien-1,2. Im selektiven Lösungsmittel weniger lösliche Kohlenwasserstoffe als Butadien-1,3 sind beispielsweise die Butane, die n-Butene, Isobuten.

Die extraktive Destillation kann unter Anwendung einer Extraktivdestillationszone durchgeführt werden. Mit besonderem Vorteil wird das Verfahren zur Gewinnung von Butadien-1,3 jedoch unter Anwendung von zwei hintereinandergeschalteten Extraktivdestillationszonen unter Verwendung des gleichen selektiven Lösungsmittels durchgeführt. Hierbei wird beispielsweise in der ersten Stufe der Extraktivdestillation ein die weniger löslichen Kohlenwasserstoffe enthaltendes Destillat (Raffinat) und ein Butadien-1,3 und die löslicheren Kohlenwasserstoffe und das selektive Lösungsmittel enthaltender Exrakt erhalten. Dieser Extrakt wird vom selektiven Lösungsmittel befreit, wobei ein Gemisch aus Butadien-1,3 und den löslicheren Kohlenwasserstoffen erhalten wird. Dieses Gemisch wird in einer zweiten extraktiven Destillationszone einer zweiten extraktiven Destillation mit dem selektiven Lösungsmittel unterworfen, wobei Butadien-1,3 als Destillat und ein Extrakt erhalten werden, der die löslicheren Kohlenwasserstoffe einschließlich der höheren Acetylene und noch zurückgebliebenes Butadien-1,3 und das selektive Lösungsmittel enthält. Der erhaltene Extrakt wird anschließend vom selektiven Lösungsmittel befreit, wobei ein die löslicheren Kohlenwasserstoffe einschließlich der $C_4$-Acetylene enthaltender Kohlenwasserstoffstrom erhalten wird.

Die Abtrennung des Propins erfolgt entweder durch Destillation des Ausgangs-$C_4$-Kohlenwasserstoffgemischs in einer der extraktiven Destillation vorgeschalteten Destillationszone oder durch Destillation des nach der extraktiven Destillation erhaltenen, Propin enthaltenden Butadien-1,3-Stromes in einer der extraktiven Destillation nachgeschalteten Destillationszone, wobei als Destillat ein Butadien-1,3 und Propin enthaltendes Kohlenwasserstoffgemisch, zweckmäßig als Kopfprodukt der Destillation, erhalten wird. Aus Sicherheitsgründen weist das Destillat im allgemeinen einen Propin-Gehalt von höchstens 70 Mol%, vorzugsweise höchstens 60 Mol%, insbesondere höchstens 50 Mol% auf.

Das aus der extraktiven Destillation vor- bzw. nachgeschalteten Destillationszone erhaltene, Propin und Butadien-1,3 enthaltende Kohlenwasserstoffgemisch wird anschließend einer weiteren Destillationszone zugeführt, und gleichzeitig wird der weiteren Destillationszone ein zweiter Strom von flüssigen oder gasförmigen Kohlenwasserstoffen zugeführt.

Als flüssige oder gasförmige Kohlenwasserstoffe, die der weiteren Destillationszone als zweiter Strom zugeführt werden, werden im allgemeinen $C_3$- und/oder $C_4$-Kohlenwasserstoffe, vorzugsweise gesättigte und/oder einfach olefinisch ungesättigte $C_3$- und/oder $C_4$-Kohlenwasserstoffe verwendet. Zweckmäßig werden diese Kohlenwasserstoffe in Form von Gemischen, z.B. als $C_3$- oder $C_4$-Kohlenwasserstoffgemische angewendet. Geeignete Kohlenwasserstoffe sind z.B. Propan, Propen, die Butane, n-Buten, Isobuten oder deren Gemische. Vorteilhaft werden $C_4$-Kohlenwasserstoffgemische eingesetzt. In einer bevorzugten Ausführungsform des Verfahrens wird das in der ersten extraktiven Destillationszone erhaltene Destillat (Raffinat) verwendet, welches die Butane und Butene enthält.

Der der weiteren Destillationszone zuzuführende zweite Strom von Kohlenwasserstoffen kann mit dem Propin und Butadien-1,3 enthaltenden Kohlenwasserstoffstrom zunächst vermischt werden und anschließend der weiteren Destillationszone zur konventionellen Destillation zugeführt werden. Vorzugsweise wird der zweite Kohlenwasserstoffstrom jedoch am oder zweckmäßig oberhalb des Zulaufpunktes des Propin und Butadien-1,3 enthaltenden Kohlenwasserstoffstromes der weiteren Destillationszone zugeführt, wobei eine Zuführung im oberen Drittel, vorteilhaft im oberen Viertel, insbesondere am Kopf der weiteren Destillationszone bevorzugt wird. Durch die Zuführung des zweiten Kohlenwasserstoffstromes oberhalb des Zulaufpunktes des Propin und Butadien-1,3 enthaltenden Kohlenwasserstoffstromes wird durch die dadurch erfolgende Gegenstromwäsche eine besonders günstige Trennung des Propin und Butadien-1,3 erzielt, so daß bei relativ neidrigem Dampfverbrauch für die Trennung ein Sumpfprodukt mit einer hohen Butadien-1,3-Konzentration erhalten werden kann. In einer bevorzugten Ausführungsform des erfindungsgemäßen Verfahrens wird der zweite Kohlenwasserstoffstrom dem flüssigen Rücklauf der Destillationszone zugemischt oder anstelle eines flüssigen Rücklaufs zugemischt.

Der Propin und Butadien-1,3 enthaltende Kohlenwasserstoffstrom wird der weiteren Destillationszone zweckmäßig in einem mittleren Bereich zugeführt, der sich von der Mitte der Destillationszone ausgehend bis jeweils zu etwa 80%, vorzugsweise zu etwa 60%, insbesondere zu etwa 50% in die obere und untere Hälfte der weiteren Destillationszone erstreckt. Der Propin und Butadien-1,3 enthaltende Kohlenwasserstoffstrom und der zweite Kohlenwasserstoffstrom können gasförmig oder flüssig der weiteren Destillationszone zugeführt werden. Zweckmäßig werden sie flüssig zugeführt. Im allgemeinen beträgt das Gewichtsverhältnis der Kohlenwasserstoffe des zweiten Kohlenwasserstoffstromes zu denen des Propin und Butadien-1,3 enthaltenden Kohlenwasserstoffstroms 10:1 bis 1:10, vorzugsweise 5:1 bis 1:5, insbesondere 2:1 bis 1:2. Für die weitere Destillationszone werden im allgemeinen herkömmliche Destillationskolonnen, z.B. Füllkörperkolonnen oder Bodenkolonnen verwendet. Durch die erfindungsgemäße Arbeitsweise wird als Kopfprodukt der weiteren Destillationszone ein das Propin enthaltendes Destillat mit einem Propingehalt von zweckmäßig höchstens 70 Mol%, vorzugsweise höchstens 60 Mol%, insbesondere höchstens 50 Mol% erhalten, welches gleichzeitig einen wesentlich geringeren Butadiengehalt aufweist, z.B. von höchstens 10 Gew.%, vorzugsweise höchstens 5 Gew.%, so daß der Butadien-1,3-Verlust bei der Butadien-1,3-Gewinnung deutlich verringert wird.

Das Sumpfprodukt der weiteren Destillationszone, welches den größten Teil des der weiteren Destillationszone zugeführten Butadien-1,3 enthält, wird in die extraktive Destillation zurückgeführt. Zweckmäßig wird das Sumpfprodukt dem der ersten extraktiven Destillationszone zugeführten Ausgangs-$C_4$-Kohlenwasserstoffgemisch zugemischt.

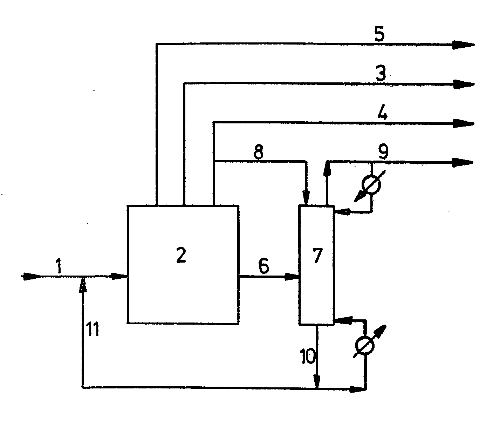

Die Figur ist ein schematisches Diagramm einer Ausführungsform des erfindungsgemäßen Verfahrens. Ein Butadien-1,3 und kleine Mengen Propin enthaltendes $C_4$-Kohlenwasserstoffgemisch ($C_4$-Fraktion aus einer Äthylenanlage) wird durch Leitung 1 in eine Butadien-Gewinnungsanlage 2 eingeführt, in der es zunächst in zwei hintereinandergeschalteten extraktiven Destillationszonen unter Verwendung eines selektiven Lösungsmittels (N-Methyl-pyrrolidon) in einen die $C_4$-Acetylene enthaltenden Strom 3, einen die Butane und Butene enthaltenden Raffinat-Strom 4 und einen Butadien-1,3-Strom aufgetrennt wird und der erhaltene Butadien-1,3-Strom danach in einer Destillationskolonne in einen als Sumpfprodukt erhaltenen Reinstbutadien-1,3-Strom 5 und einen als Kopfprodukt der Destillationskolonne erhaltenen Propin/Butadien-1,3-Strom 6 weiter aufgetrennt wird. Der Propin/Butadien-1,3-Strom wird in das mittlere Drittel einer Füllkörperkolonne 7 eingeleitet, während aus dem Raffinat-Strom 4 ein kleiner Teilstrom 8 abgezweigt und auf den Kopf von Kolonne 7 gegeben wird. Am Kopf der Kolonne 7 wird ein das Propin enthaltender Kohlenwasserstoffstrom 10 abgezogen, der nur noch einen geringen Butadien-1,3-Gehalt aufweist. Am Sumpf der Kolonne 7 wird ein nur noch geringe Spuren Propinenthaltender Kohlenwasserstoffstrom 10 mit einem hohen Butadien-1,3-Gehalt abgezogen, der durch Leitung 11 in die extraktive Destillation zurückgeführt wird.

Die nachstehenden Beispiele dienen der weiteren Erläuterung der Erfindung.

### Beispiel 1

Aus einer Butadien-1,3-Gewinnung bestehend aus zwei hintereinandergeschalteten Extraktivdestillationen und einer nachgeschalteten Destillationskolonne wird als Kopfprodukt der Destillationskolonne ein Gemisch aus 50 Vol% Butadien-1,3 und 50 Vol% Propin erhalten.

100g/h dieses Propin/Butadien-1,3-Gemisches werden am 28. Boden einer Kolonne mit 45 Böden eingeleitet. Am Kopf der Kolonne werden 80g/h Raffinat aus der Butadien-1,3-Gewinnung zugeführt, das im wesentlichen aus Butan, iso-Butan, Buten-1, iso-Buten, Buten-2-trans, Buten-2-cis, Propan, Propen besteht. Am Sumpf der Kolonne wird soviel Wärme zugeführt, daß ein Rücklauf von 99,2g/h erreicht wird. Am Kopf der Kolonne werden 102g/h eines nur 5,7 Gew.% Butadien-1,3 und 41,7 Gew.% Propin enthaltenden Kohlenwasserstoffstromes abgezogen. Am Sumpf werden 78g/h eines Kohlenwasserstoffstromes aus 66,4 Gew.% Butadien-1,3 und 33,6 Gew.% eines Gemisches aus $C_3$- und $C_4$-Kohlenwasserstoffen abgezogen, das in die Extraktivdestillation zurückgeführt wird. Das Sumpfprodukt enthält nur 36 Gew.-ppm Propin. An keiner Stelle der Kolonne wird eine Propinkonzentration von 50 Vol% überschritten. Diese Grenze war vorgesehen, um einen genügenden Abstand von der Selbstzersetzungsgrenze des Propins einzuhalten.

### Beispiel 2

Arbeitet man wie in Beispiel 1 beschrieben, steigert jedoch die Zulaufmenge des Raffinats auf 150g/h und die Rücklaufmenge auf 180g/h, so läßt sich der Butadiengehalt im Kopfprodukt auf 1 Gew.% senken.

Am Sumpf werden 148g/h eines Gemisches aus 38,4 Gew.% Butadien-1,3 und 61,6 Gew.% $C_3$- und $C_4$-Kohlenwasserstoffen abgezogen. Propin ist im Sumpfprodukt zu 115 Gew.-ppm enthalten und liegt in der gesamten Kolonne in Konzentrationen von weniger als 50 Vol.% vor.

**Patentansprüche**

1. Verfahren zur Gewinnung von Butadien-1,3 mit Hilfe eines selektiven Lösungsmittels aus einem $C_4$-Kohlenwasserstoffgemisch, welches Butadien-1,3, Propin, im selektiven Lösungsmittel löslichere Kohlenwasserstoffe als Butadien-1,3 und im selektiven Lösungsmittel weniger lösliche Kohlenwasserstoffe als Butadien-1,3 enthält, bei dem das $C_4$-Kohlenwasserstoffgemisch unter Anwendung von einer oder mehreren extraktiven Destillationszonen in ein die weniger löslichen Kohlenwasserstoffe enthaltendes Destillat, einen Strom aus Butadien-1,3 und einen die löslicheren Kohlenwasserstoffe enthaltenden Strom aufgetrennt wird und das Propin entweder durch Destillation des $C_4$-Kohlenwasserstoffgemischs in einer der bzw. den extraktiven Destillationszonen vorgeschalteten Destillationszone oder durch Destillation des aus der bzw. den extraktiven Destillationszonen erhaltenen Butadien-1,3-Stromes in einer nachgeschalteten Destillationszone abgetrennt wird, wobei ein Propin und Butadien-1,3 enthaltender Kohlenwasserstoffstrom erhalten, wird, *dadurch gekennzeichnet,* daß der Propin und Butadien-1,3 enthaltende Kohlenwasserstoffstrom einer weiteren Destillationszone zugeführt wird, wobei gleichzeitig ein zweiter Strom von flüssigen oder gasformigen Kohlenwasserstoffen der weiteren Destillationszone zugeführt wird, und das Sumpfprodukt der weiteren Destillationszone in die extraktive Destillation zurückgeführt wird.

2. Verfahren nach Anspruch 1, *dadurch gekennzeichnet,* daß der weiteren Destillationszone als zweiter Strom ein Gemisch aus $C_3$- und/oder $C_4$-Kohlenwasserstoffen zugeführt wird.

3. Verfahren nach Anspruch 1 und 2, *dadurch gekennzeichnet,* daß der weiteren Destillationszone als zweiter Strom ein gesättigter oder einfach olefinisch ungesättigter $C_4$-Kohlenwasserstoff bzw. ein Gemisch aus gesättigten und/oder einfach olefinisch ungesättigten $C_4$-Kohlenwasserstoffen zugeführt wird.

4. Verfahren nach Anspruch 1 bis 3, *dadurch gekennzeichnet,* daß der zweite Kohlenwasserstoffstrom am oder oberhalb des Zulaufpunktes des Propin und Butadien-1,3 enthaltenden Kohlenwasserstoffstroms der weiteren Destillationszone zugeführt wird.

5. Verfahren nach Anspruch 1 bis 4, *dadurch gekennzeichnet,* daß der zweite Kohlenwasserstoffstrom im oberen Drittel der weiteren Destillationszone zugeführt wird.

## Revendications

1. Procédé de préparation de butadiène-1,3 à l'aide d'un solvant sélectif, à partir d'un mélange d'hydrocarbures en $C_4$ contenant du butadiène-1,3, du propyne, des hydrocarbures plus solubles dans le solvant sélectif que le butadiène-1,3, et des hydrocarbures moins solubles dans le solvant sélectif que le butadiène-1,3, dans lequel le mélange d'hydrocarbures en $C_4$, dans une ou plusieurs zones de distillation extractive, est séparé en un distillat contenant les hydrocarbures moins solubles, un courant de butadiène-1,3 et un courant contenant les hydrocarbures plus solubles, et le propyne est séparé, soit par distillation du mélange d'hydrocarbures en $C_4$ dans une zone de distillation précédant la zone ou les zones de distillation extractive, soit par distillation, dans une zone de distillation montée à la suite, du courant de butadiène-1,3 venant de la zone ou des zones de distillation extractive, avec obtention d'un courant d'hydrocarbures contentant du propyne et du butadiène-1,3, ce procédé étant caractérisé par le fait que l'on amène le courant d'hydrocarbures contenant du propyne et du butadiène-1,3 à une zone de distillation ultérieure dans laquelle on introduit en même temps un deuxiéme courant d'hydrocarbures liquides ou gazeux, et on ramène vers la distillation extractive le produit contenu dans la nappe liquide de la zone de distillation ultérieure.

2. Procédé selon la revendication 1, dans lequel on amène à la zone de distillation ultérieure, comme deuxième courant, un mélange d'hydrocarbures en $C_3$ et/ou $C_4$.

3. Procédé selon l'une des revendications 1 et 2, dans lequel on amène à la zone distillation ultérieure, comme deuxième courant, un hydrocarbure en $C_4$ saturé ou oléfiniquement mono-insaturé ou un mélange d'hydrocarbures en $C_4$ saturés et/ou oléfiniquement mono-insaturés.

4. Procédé selon l'une des revendications 1 à 3, dans lequel on amène le deuxième courant d'hydrocarbures à la zone de distillation ultérieure au point d'admission ou en amont du point d'admission du courant d'hydrocarbures contenant du propyne et du butadiène-1,3.

5. Procédé selon l'une des revendications 1 à 4 dans lequel on amène le deuxième courant d'hydrocarbures au tiers supérieur de la zone de distillation ultérieure.

## Claims

1. A process for isolating 1,3-butadiene by means of a selective solvent from a $C_4$-hydrocarbon mixture which contains 1,3-butadiene, propyne, hydrocarbons which are more soluble than 1,3-butadiene in the selective solvent and hydrocarbons which are less soluble than 1,3-butadiene in the selective solvent, wherein the $C_4$-hydrocarbon mixture is separated, using one or more extractive distillation zones, into a distillate containing the less soluble hydrocarbons, a stream of 1,3-butadiene and a stream containing the more soluble hydrocarbons, and the propyne is separated off either by distillation of the $C_4$-hydrocarbon mixture in a distillation zone upstream of the extractive distillation zone or zones or by distillation of the stream of 1,3-butadiene, obtained from the extractive distillation zone or zones, in a downstream distillation zone, giving a stream of hydrocarbons containing propyne and 1,3-butadiene, *characterized in that* the stream of hydrocarbons containing propyne and 1,3-butadiene is fed to an additional distillation zone, whilst at the same time a second stream of liquid or gaseous hydrocarbons is fed to the additional distillation zone, and the bottom product from the additional distillation zone is recycled to the extractive distillation.

2. A process as claimed in claim 1, *characterized in that* a mixture of $C_3$- and/or $C_4$-hydrocarbons is fed, as the second stream, to the additional distillation zone.

3. A process as claimed in claim 1 or 2, *characterized in that* a saturated or monoolefinically unsaturated $C_4$hydrocarbon, or a mixture of saturated and/or monoolefinically unsaturated $C_4$-hydrocarbons, is fed as the second stream to the additional distillation zone.

4. A process as claimed in any of claims 1 to 3, *characterized in that* the second hydrocarbon stream is fed to the additional distillation zone at or above the feed point of the hydrocarbon stream containing propyne and/or 1,3-butadiene.

5. A process as claimed in any of claims 1 to 4, *characterized in that* the feed point of the second hydrocarbon stream is in the upper third of the additional distillation zone.